(19) Europäisches Patentamt
European Patent Office
Office européen des brevets

(11) **EP 3 984 494 B1**

(12) **EUROPÄISCHE PATENTSCHRIFT**

(45) Veröffentlichungstag und Bekanntmachung des Hinweises auf die Patenterteilung:
**29.01.2025 Patentblatt 2025/05**

(21) Anmeldenummer: **20215945.5**

(22) Anmeldetag: **21.12.2020**

(51) Internationale Patentklassifikation (IPC):
*A61C 5/73* (2017.01)    *A61C 5/77* (2017.01)
*A61C 13/00* (2006.01)    *A61C 13/08* (2006.01)
*B33Y 80/00* (2015.01)    *B33Y 50/00* (2015.01)

(52) Gemeinsame Patentklassifikation (CPC):
**A61C 5/77; A61C 5/73; A61C 13/0004;
A61C 13/08; A61C 13/082; A61C 13/09;
B33Y 50/00; B33Y 80/00**

(54) **VERFAHREN ZUM HERSTELLEN EINER DENTALEN RESTAURATION**

METHOD FOR PRODUCING A DENTAL RESTORATION

PROCÉDÉ DE RÉALISATION D'UNE RESTAURATION DENTAIRE

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **13.10.2020 EP 20201553**

(43) Veröffentlichungstag der Anmeldung:
**20.04.2022 Patentblatt 2022/16**

(73) Patentinhaber: **Ivoclar Vivadent AG
9494 Schaan (LI)**

(72) Erfinder:
• **JUSSEL, Rudolf
6800 Feldkirch (AT)**
• **JOHN, Hendrik
9470 Buchs (CH)**
• **NIEDRIG, Christian
9464 Rüthi (CH)**

(74) Vertreter: **Baldus, Oliver
Splanemann
Rumfordstrasse 7
80469 München (DE)**

(56) Entgegenhaltungen:
**WO-A1-2019/023461    US-A1- 2012 205 828**

**Beschreibung**

Die vorliegende Erfindung betrifft ein Verfahren zum Herstellen einer dentalen Restauration, eine Computervorrichtung zum Herstellen einer dentalen Restauration und ein Computerprogramm.

Bei analogen und digitalen Fertigungsprozessen von dentalen Restaurationen erfolgt die Auswahl der Restaurationsmaterialen, wie deren Farbe, subjektiv durch einen Anwender unter Zuhilfenahme eines analogen Farbschlüssels. Danach folgt ein schichtweiser manueller Restaurationsaufbau. Ein Restaurationsaufbau erfolgt durch Übernahme eines Zahnaufbaus aus dem natürlichen Vorbild. Dieser kann in Datenbanken oder Bibliotheken bereitgestellt werden. Alternativ führt der Zahntechniker den Schichtaufbau basierend auf seiner Fachexpertise manuell oder CAD-gestützt aus. Diese Prozesse sind ungenau und geben das gewünschte natürliche Erscheinungsbild oft nur unzureichend wieder. Zudem bleibt es nur erfahrenen Zahntechnikern vorbehalten ein natürliches Erscheinungsbild zu erzielen.

Die Druckschrift WO2019/023461 A1 betrifft ein Verfahren zum Herstellen einer Zahnrestauration für einen Patienten, sodass die Zahnrestauration optische Eigenschaften aufweist, die den optischen Eigenschaften der Zähne des Patienten entsprechen.

US 2012/205828 A1 offenbart ein Verfahren zum Herstellen einer Zahnrestauration, wobei eine Schichtdicke der Zahnrestauration sowie eine Materialauswahl angepasst wird nach einem optischen Vergleich der auf einem Bildschirm dargestellten gerenderten Zahnrestauration mit einem Bild eines Nachbarzahns.

Es ist die technische Aufgabe der vorliegenden Erfindung, einen internen Aufbau für eine dentale Restauration derart zu bestimmen, dass diese einem gewünschten natürlichen Erscheinungsbild entspricht.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Verfahren zum Herstellen einer dentalen Restauration gelöst, mit den Schritten eines Erzeugens eines digitalen Zahnmodells mit einem ersten räumlichen Bereich, in dem ein erstes Restaurationsmaterial angeordnet ist, und einem zweiten räumlichen Bereich, in dem ein zweites Restaurationsmaterial angeordnet ist; eines Renderns des digitalen Zahnmodells zum Erzeugen eines Ist-Datensatzes, der die optischen Eigenschaften des digitalen Zahnmodells wiedergibt; eines Bestimmens einer Abweichung zwischen einem Soll-Datensatz und dem Ist-Datensatz; eines Veränderns des ersten räumlichen Bereichs und/oder zweiten räumlichen Bereichs, um eine geringere Abweichung zwischen dem erfassten Soll-Datensatz und dem Ist-Datensatz des erneut gerenderten digitalen Zahnmodells zu erhalten; und eines Herstellens der dentalen Restauration auf Basis des digitalen Zahnmodells, mit der geringeren Abweichung. Der erste und der zweite räumliche Bereich bilden ein Subvolumen des Zahnmodells. Die Restaurationsmaterialien der räumlichen Bereiche können im Laufe des Verfahrens unverändert beibehalten werden.

Durch das Verfahren kann die innere Architektur einer dentalen Restauration automatisch derart gestaltet werden, dass sich ein Gesamteindruck ergibt, der sich in das Zahngesamtbild des Patienten einfügt. Zudem können subjektive Einflüsse eines Anwenders auf den optischen Gesamteindruck der dentalen Restauration ausgeschlossen werden und eine hohe Genauigkeit und Wiederholbarkeit bei der Herstellung der dentalen Restauration erzielt werden.

In einer technisch vorteilhaften Ausführungsform des Verfahrens erfolgt das Verändern derart, dass der erste räumliche Bereich ein äußerer Bereich des Zahnmodells ist, der nach Innen verformt wird. Von der Außenfläche kann ein zweiter räumlicher Bereich nach innen abgeleitet und verformt werden. Dabei wird eine neue Grenzfläche innerhalb des ersten räumlichen Bereichs erzeugt, die einen definierten Abstand zur Außenfläche hat. Dieser Abstand kann punktuell verändert werden. Dadurch wird beispielsweise der technische Vorteil erreicht, dass das Verfahren in einem weiten Raumbereich ausgeführt wird und gute Ergebnisse erhalten werden.

In einer weiteren technisch vorteilhaften Ausführungsform des Verfahrens erfolgt das Verändern derart, dass der zweite räumliche Bereich ein innerer Bereich des Zahnmodells ist, der nach Außen verformt wird. Dadurch wird beispielsweise ebenfalls der technische Vorteil erreicht, dass das Verfahren in einem weiten Raumbereich ausgeführt wird und gute Ergebnisse erhalten werden.

In einer weiteren technisch vorteilhaften Ausführungsform des Verfahrens wird das digitale Zahnmodells mit einer Grenzfläche zwischen dem ersten räumlichen Bereich und dem zweiten räumlichen Bereich erzeugt. Dadurch wird beispielsweise der technische Vorteil erreicht, dass die Grenzfläche verschoben werden kann, um gleichzeitig den ersten und den zweiten räumlichen Bereich zu verändern.

In einer weiteren technisch vorteilhaften Ausführungsform des Verfahrens liegt die Grenzfläche in einer Mitte des digitalen Zahnmodells. Dadurch wird beispielsweise der technische Vorteil erreicht, dass das Verfahren schnell zu einer geringen Abweichung hin konvergiert.

In einer weiteren technisch vorteilhaften Ausführungsform des Verfahrens verläuft die Grenzfläche tangential zum Zahnbogen an der entsprechenden Zahnposition. Dadurch wird beispielsweise der technische Vorteil erreicht, dass das Verfahren noch schneller zu einer zu einer geringen Abweichung hin konvergiert.

In einer weiteren technisch vorteilhaften Ausführungsform des Verfahrens liegt die Grenzfläche an einer Innenseite oder einer Außenseite des digitalen Zahnmodells. Dadurch wird beispielsweise der technische Vorteil erreicht, dass das Zahnmodell von Innen oder außen her verändert werden kann.

In einer weiteren technisch vorteilhaften Ausführungsform des Verfahrens wird durch die Grenzfläche ein Keimbereich gebildet, von dem ausgehend der erste räumliche Bereich und/oder der zweite räumliche Bereich verändert werden. Dadurch wird beispielsweise der technische Vorteil erreicht, dass ein Subvolumen gebildet wird, von dem aus sich die räumlichen Bereiche ausdehnen können.

In einer weiteren technisch vorteilhaften Ausführungsform des Verfahrens sind der erste räumliche Bereich und der zweite räumliche Bereich schichtweise zueinander angeordnet. Dadurch wird beispielsweise der technische Vorteil erreicht, dass ein natürlicher Zahnaufbau nachgeahmt wird und sich mit wenigen Schritten eine geringe Abweichung erzielen lässt.

Im beanspruchten Verfahren wird der erste räumliche Bereich und/oder zweite räumliche Bereich solange verformt, bis die Abweichung zwischen Soll- und Ist Datensatz unter einem vorgegebenen Wert liegt. Dadurch wird beispielsweise der technische Vorteil erreicht, dass die dentale Restauration mit einer vorbestimmten Genauigkeit hergestellt werden kann.

In einer weiteren technisch vorteilhaften Ausführungsform des Verfahrens wird der erste räumliche Bereich und/oder zweite räumliche Bereich solange verändert, bis die Abweichung zwischen Soll- und Ist Datensatz ein Minimum erreicht hat. Dadurch wird beispielsweise der technische Vorteil erreicht, dass die dentale Restauration mit einer optimalen Anpassung an den Soll-Datensatz hergestellt werden kann.

In einer weiteren technisch vorteilhaften Ausführungsform des Verfahrens wird der Soll-Datensatz auf Basis eines natürlichen Zahns erhalten. Dadurch wird beispielsweise der technische Vorteil erreicht, dass die dentale Restauration an einen natürlichen Zahn angepasst werden kann.

In einer weiteren technisch vorteilhaften Ausführungsform des Verfahrens gibt der Soll-Datensatz die optischen Eigenschaften und/oder die Geometrie des natürlichen Zahns wieder. Dadurch wird beispielsweise der technische Vorteil erreicht, dass die Wiedergabetreue der dentalen Restauration nochmals verbessert wird.

Gemäß einem zweiten Aspekt wird die technische Aufgabe durch eine Computervorrichtung zum Herstellen einer dentalen Restauration gelöst, mit einer Herstellungsvorrichtung, die geeignet ist, das Verfahren nach dem ersten Aspekt auszuführen. Dadurch werden die gleichen technischen Vorteile wie durch das Verfahren nach dem ersten Aspekt erreicht.

Gemäß einem dritten Aspekt wird die technische Aufgabe durch ein Computerprogramm gelöst, umfassend Befehle, die bewirken, dass die Computervorrichtung nach dem zweiten Aspekt die Verfahrensschritte nach dem ersten Aspekt ausführt. Dadurch werden die gleichen technischen Vorteile wie durch das Verfahren nach dem ersten Aspekt erreicht.

Ausführungsbeispiele der Erfindung sind in den Zeichnungen dargestellt und werden im Folgenden näher beschrieben.

Es zeigen:

Fig. 1     eine schematische Darstellung eines digitalen Zahnmodells;

Fig. 2     ein Verändern der räumlichen Bereiche eines Zahnmodells;

Fig. 3     eine Darstellung eines Vergleichs zwischen einem Soll-Datensatz und einem Ist-Datensatz;

Fig. 4     eine schematische Darstellung zur Berechnung einer Abweichung zwischen einem Soll-Datensatz und einem Ist Datensatz; und

Fig. 5     ein Blockdiagramm eines Verfahrens zum Herstellen einer dentalen Restauration.

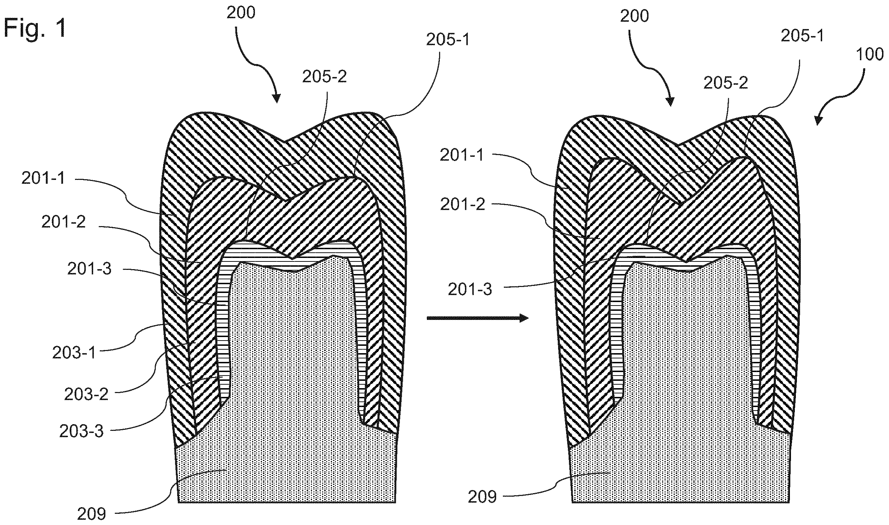

Fig. 1 zeigt eine schematische Darstellung eines digitalen Zahnmodells 200, das auf einen Restzahn 209 aufgesetzt wird. Durch das digitale Zahnmodelle 200 wird die äußere Form und der innere räumliche Aufbau der späteren dentalen Restauration 100 vorgegeben. Die äußere Form kann hierzu in einem CAD-Programm erzeugt werden. Durch die Modellierung der dentalen Restauration 100 im CAD-Programm wird die äußere Form festgelegt.

Der innere räumliche Aufbau des Zahnmodells 200 kann errechnet oder aus Datenbanken entnommen werden, beispielsweise abgeleitet vom Aufbau eines natürlichen Zahns oder aus der äußeren Form der dentalen Restauration 100 oder eines natürlichen Zahns errechnet werden. Bei vorgegebener äußerer Form der dentalen Restauration 100 kann somit durch das Zahnmodell 200 ein innerer Schichtaufbau festgelegt werden und jeder einzelnen Schicht 203-1, 203-2, und 203-3 ein eigenes Restaurationsmaterial 201-1, 201-2 und 201-3 mit vorgegebenen optischen Materialparametern zugewiesen werden. Zwischen den Restaurationsmaterialien 201-1, 201-2, und 201-3 befinden sich die Grenzflächen 205-1 und 205-2. Die unterschiedlichen Restaurationsmaterialien 201-1, ..., 201-3 in den räumlichen Bereichen 203-1, ...,

203-3 sind durch die Grenzflächen 205-1 und 205-2 voneinander getrennt.

Das Zahnmodell 200 gibt beispielsweise eine dentale Restauration 100 wieder, die schichtweise aus den drei unterschiedlichen Restaurationsmaterialen 201-1, 201-2 und 201-3 aufgebaut ist. In einer äußeren Schicht 203-1 wird das Restaurationsmaterial 201-1 verwendet, in einer mittleren Schicht 203-2 wird das Restaurationsmaterial 201-2 verwendet und in einer inneren Schicht 203-3 wird das Restaurationsmaterial 201-3 verwendet. Die optischen und physikalischen Eigenschaften der jeweils zugewiesenen Restaurationsmaterialien 201-1, ..., 201-3 sind bekannt, wie beispielsweise Farbwerte, Streuwerte, Reflexionswerte, Transmissionswerte und/oder Absorptionswerte.

Sind die äußere Form und der innere Aufbau des Zahnmodells 200 mit den unterschiedlichen Restaurationsmaterialien 201-1, ..., 201-3 bekannt, kann hieraus das spätere Aussehen der dentalen Restauration 100 errechnet werden. Durch ein Rendering (Lichtsimulationsverfahren) mittels Strahlverfolgung (Ray Tracing) werden die Farbe und die Transluzenz der dentalen Restauration 100 auf Basis des erstellten Zahnmodells 200 berechnet. Auf diese Weise kann aus dem Zahnmodell 200 das spätere Aussehen und der optische Eindruck des biomimetischen Zahnersatzes präzise errechnet werden, wie beispielsweise einer Krone, Teilkrone, Inlay, Onlay oder einem Veneer.

Das Rendern wird mittels einer physikalisch korrekten Simulation der Wechselwirkung von Licht mit den verwendeten Restaurationsmaterialien 201-1, ..., 201-3 durchgeführt. Dabei werden die bekannten optischen Parameter der einzelnen Restaurationsmaterialien 201-1, ..., 201-3 verwendet, um eine computergestützte Ansicht der dentalen Restauration 100 zu erzeugen.

Hierzu kann zusätzlich bestehendes natürliches Zahnmaterial berücksichtigt werden, wie beispielsweise ein Restzahn 209, auf dem die dentale Restauration 100 aufgesetzt werden soll. Beim Rendern wird daher zu dem vorgegebenen inneren Aufbau und den ausgewählten Restaurationsmaterialien 201-1, ..., 201-3 der optische Eindruck der späteren dentalen Restauration 100 errechnet. Für das Rendern kann computergestützt eine Berechnung von Reflexions-, Transmissions- und Absorptionswerten im sichtbaren Bereich bei mindestens drei Wellenlängen durchgeführt werden.

Für das Herstellungsverfahren der dentalen Restauration 100 wird zunächst ein Soll-Datensatz ermittelt. Der Soll-Datensatz kann durch ein optisches Erfassen und Auswerten eines benachbarten Zahns erhalten werden. Hierzu kann eine elektronische Kamera oder ein 3D-Scanner verwendet werden, mit dem die Farbwerte, Reflexions-, Transmissions- und/oder Absorptionswerte und die räumliche Form oder ein Bild des natürlichen Zahns bestimmt werden können. Daneben kann auch eine Farbmessung oder Transluzenz-Messung mit einem Transluzenz-Schema basierend auf den acht natürlichen Farbräumen durchgeführt werden. Auf Basis dieser Daten wird die dentale Restauration 100 mit möglichst identischen Eigenschaften geplant.

Hierzu wird ein anfängliches Zahnmodell 200 für die dentale Restauration 100 erstellt, mit dem die äußere Form und die inneren räumlichen Bereiche 203-1, ..., 203-3 des Zahnmodells 200 mit den zugewiesenen Restaurationsmaterialien 201-1, 201-2 und 201-3 festgelegt werden. Auf Basis dieses anfänglichen Zahnmodells 200 wird das optische Erscheinungsbild der dentalen Restauration 100 gerendert, um einen Ist-Datensatz zu erzeugen, der die optischen Eigenschaften wiedergibt.

Idealerweise wird das Rendern der dentalen Restauration 100 aus demselben Blickwinkel oder derselben Perspektive durchgeführt, aus der der Soll-Datensatz auf Basis des natürlichen Zahns gewonnen worden ist. Zur Verbesserung der Ergebnisse kann das Rendern auch aus verschiedenen Blickwinkeln erfolgen. Das Rendern kann für jeden beliebigen Blickwinkel und beliebig wählbare Umgebungssituationen erfolgen, wie beispielsweise einer vorgegebenen Beleuchtungssituation, unter Berücksichtigung von Nachbarzähnen, einer Position der dentalen Restauration 100 im Mundraum oder einer Form und optischen Eigenschaften des zu präparierenden Zahnrestes. Beim Rendern können auch weitere beeinflussende Bedingungen berücksichtigt werden, wie beispielsweise bekannte optische Daten einer Zement-, Komposit- und/oder Adhäsiv-Schicht.

Anschließend wird ein Vergleich (Map Matching) des gerenderten Ist-Bildes gemäß dem Ist-Datensatz mit dem aufgenommenen Soll-Bild gemäß dem Soll-Datensatz durchgeführt und eine Abweichung ermittelt, beispielsweise eine mittlere Abweichung oder Minimal- oder Maximalwerte. Die Abweichung ist ein numerischer Wert, der die Unterschiedlichkeit des Ist-Datensatzes und des Soll-Datensatzes quantifiziert.

Um das spätere Aussehen der dentalen Restauration 100 weiter anzupassen, können die räumlichen Bereiche 203-1, ..., 203-3 des Zahnmodells 200 verändert werden. Hierbei können sowohl die Grenzflächen 205-1 und 205-2 als auch die Subvolumina der einzelnen räumlichen Bereiche 203-1, ..., 203-3 verändert werden. Bei großen Abweichungen können nicht nur die räumlichen Bereiche 203-1, ..., 203-3 sondern zusätzlich auch das verwendete Restaurationsmaterial 201-1, ..., 201-3 angepasst werden. Durch die Anpassung der räumlichen Bereiche 203-1, ..., 203-3 kann beispielsweise eine Änderung der Topografie der Grenzfläche 205-1 zwischen Schneide und Dentin unter Beibehaltung der äußeren Form des Zahnmodells 200 erfolgen.

Danach erfolgt ein erneutes Rendern des veränderten Zahnmodells 200 und ein erneuter Abgleich zwischen dem gerenderten Ist-Datensatz und dem aufgenommenen Soll-Datensatz, um eine neue Abweichung zu bestimmen. Die Schritte des Veränderns des Zahnmodells und des Bestimmens einer Abweichung werden dann solange wiederholt, bis die Abweichung unter einen vorgegebenen Wert liegt.

Hierdurch erfolgt eine iterative Anpassung des Zahnmodells 200, indem die räumlichen Bereiche 203-1, ..., 203-3 angepasst werden. Die Anpassung der inneren Architektur des Zahnmodells 200 wird zur Optimierung des gesamtheitlichen optischen Erscheinungsbildes der dentalen Restauration 100 durchgeführt.

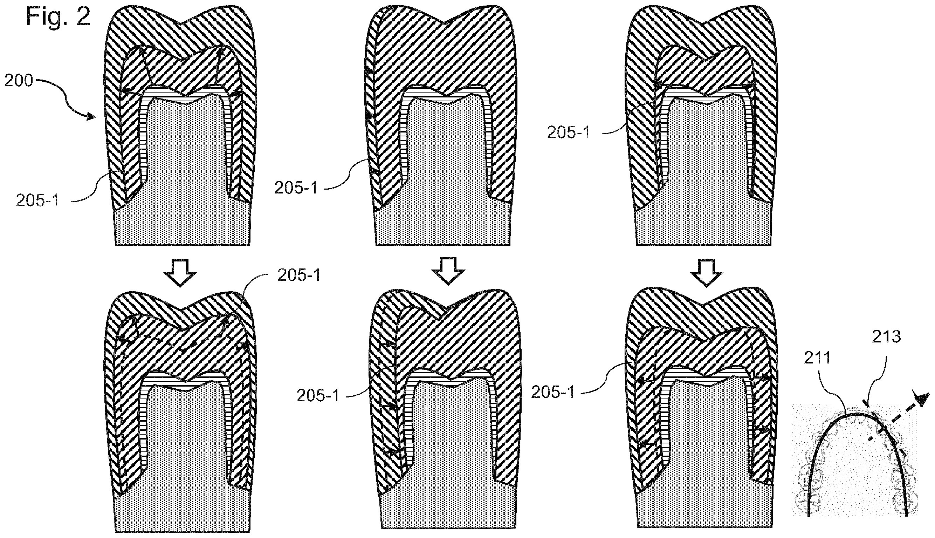

Fig. 2 zeigt ein Verändern der räumlichen Bereiche des Zahnmodells 200 im Rahmen des Herstellungsverfahrens.

In einem zunächst homogenen Volumen des Zahnmodells 200 werden Keimflächen für die Entstehung von zusätzlichen inneren Schichten definiert. Die Keimflächen können an beliebigen Positionen innerhalb des Volumens des Zahnmodells 200 angeordnet sein, beispielsweise an einer Grenzfläche zum Zahnsubstrat (Präparation), an einer labialen Oberfläche der dentalen Restauration, in einer Mittelebene 213 tangential zum Zahnbogen 211 oder einer rotationssymmetrischen Einhüllenden der senkrechten Mittelachse des Zahnmodells 200.

Ausgangspunkt ist eine homogene Verteilung des Restaurationsmaterials, wie beispielsweise ein künstliches Dentinmaterial, im räumlichen Bereich der gesamten dentalen Restauration 100. Ausgehend von einer labialen Oberfläche (Schneide) des ersten räumlichen Bereichs wird ein zweiter räumlicher Bereich gebildet, der in das Innere der dentalen Restauration 100 verformt wird und später die Schneidenschicht bildet. Diesem zweiten räumlichen Bereich ist ein Restaurationsmaterial mit anderen optischen und physikalischen Eigenschaften zugeordnet. Der zweite räumliche Bereich weist Grenzfläche 205-1 zu dem ersten räumlichen Bereich auf.

Die Keimschicht kann auch von einer Oberfläche des Zahnmodells 100 ausgehen, um in einem homogenen Bereich aus Schmelzmaterial eine neue Schicht aus Dentinmaterial wachsen zu lassen. Die Keimschicht kann sich im Zahnmodell 200 auch räumlich auf einer Mittelebene 213 befinden, beispielsweise tangential zum Zahnbogen 211 an der entsprechenden Zahnposition.

Es kann auch ein Zahnmodell 200 mit vorgegebener äußerer Form und innerer Architektur aus mehreren Schichten vorgegeben sein. In diesem Fall können die Grenzflächen 205 der Schichten iterativ dreidimensional verformt werden, um das gesamtheitliche optische Erscheinungsbild gezielt dem optischen Soll-Zustand anzunähern.

Ausgehend von einer neu entstandenen Grenzfläche 205 können weitere zusätzliche Schichten nach demselben Verfahren hinzugefügt werden. Die Verformung der jeweiligen Grenzflächen 205 erfolgt dabei durch einen iterativen Abgleich des Soll-Datensatzes und des Ist-Datensatzes solange, bis ein vorgegebener Grenzwert erreicht wird.

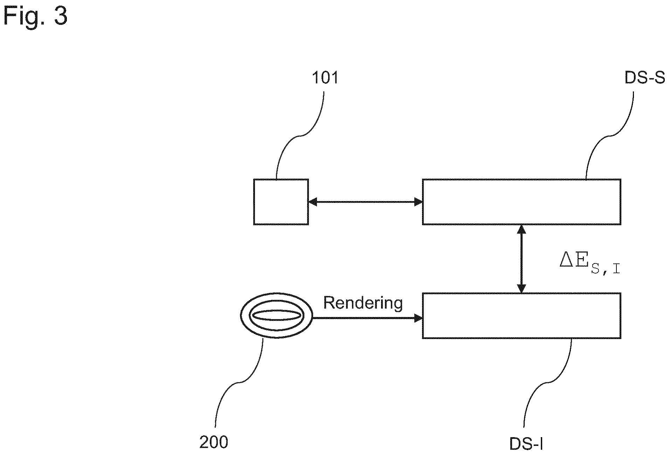

Fig. 3 zeigt eine schematische Darstellung einer Abweichung $\Delta E_{S,I}$ zwischen dem Soll-Datensatz DS-S und dem Ist-Datensatz DS-I. Der Soll-Datensatz DS-S wird beispielsweise durch eine elektronische Kamera 101 erhalten.

Das digital erzeugte Zahnmodell 200 umfasst Daten über die räumliche Geometrie der dentalen Restauration 100 und die zugeordneten Restaurationsmaterialien 201-1, ..., 201-3, aus denen die dentale Restauration 100 hergestellt werden soll und die räumlichen Bereiche 203-1, 203-2 und 203-3, in denen die Restaurationsmaterialien 201-1, ..., 201-3 angeordnet sind. Die optischen und physikalischen Eigenschaften der Restaurationsmaterialien 201-1, ..., 201-3, die für das Rendern erforderlich sind, sind in der Rendering-Software bekannt. Diese können aus einer Parametertabelle entnommen werden, die ständig um neue Materialien ergänzt wird.

Der Ist-Datensatz DS-I wird erhalten, indem das digitale Zahnmodell 200 mit einer ausgewählten Materialkombination dem Rendering unterzogen wird. Beim Rendering werden die räumliche Geometrie der dentalen Restauration 100 und die optischen und physikalischen Eigenschaften der verschiedenen, vorkommenden Restaurationsmaterialien 201-1, ..., 201-3 berücksichtigt. Es kann zusätzlich ein Klebematerial und ein Stumpf berücksichtigt werden.

Die Ausbreitung von Licht in dem Zahnmodell 200 kann durch die Maxwell-Gleichungen beschrieben werden. Das Rendering verwendet beispielsweise die Strahlungstransportgleichung (RTE - Radiative Transport Equation), bei der ein Ausbreitungsmedium durch den Absorptionskoeffizienten, den Streukoeffizienten, den Brechungsindex und die Streuphasenfunktion beschrieben wird.

Das digitale Zahnmodell 200 umfasst die Daten über die räumliche Geometrie der dentalen Restauration 100 und die innere Architektur sowie den Absorptionskoeffizienten, den Streukoeffizienten, den Brechungsindex und die Streuphasenfunktion für die jeweiligen Restaurationsmaterialien 201-1, ..., 201-3. Die Streuphasenfunktion kann beim Rendern auf Basis des Zahnmodells 200 mit den genannten Parametern in einer Monte-Carlo-Simulation in jeder gewünschten Genauigkeit numerisch gelöst werden, bei der eine Vielzahl von Photonen auf zufälligen Pfaden durch das Zahnmodell 200 propagieren.

Daraus lässt sich durch das Rendern ein Ist-Datensatz DS-I für das Aussehen der dentalen Restauration 100 errechnen, der die verwendeten Materialien, die äußere Form und die innere Architektur des Zahnmodells 200 berücksichtigt. Dieser berechnete Ist-Datensatz DS-I kann anschließend mit dem Soll-Datensatz DS-S, der auf Basis eines Nachbarzahns gewonnen worden ist, verglichen werden. Zur Vereinfachung des Vergleichs kann dieser in einer zweidimensionalen Ableitung (zweidimensionales Bild) erfolgen. Im Allgemeinen können jedoch auch dreidimensionale Verfahren verwendet werden. Als Maß für die Abweichung $\Delta E_{S,I}$ zwischen dem Ist-Datensatz DS-I und dem Soll-Datensatz DS-S wird ein numerischer Wert berechnet.

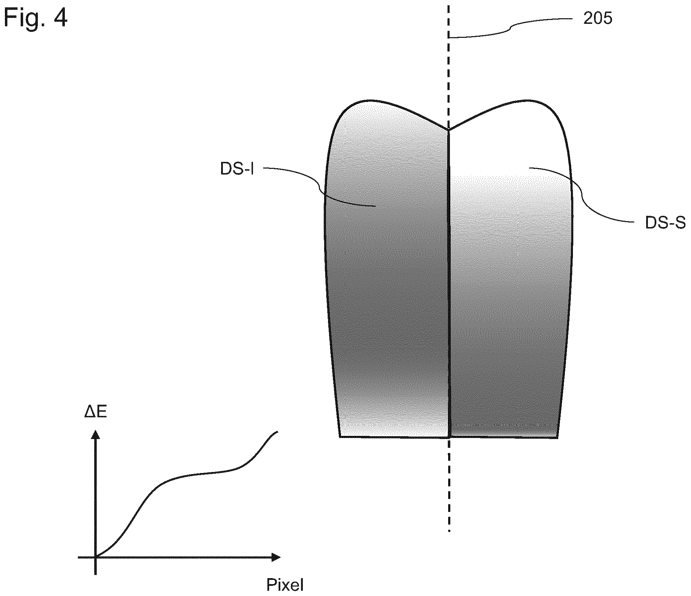

Fig. 4 zeigt eine schematische Darstellung zur Berechnung einer Abweichung $\Delta E_{S,I}$ zwischen dem Soll-Datensatz DS-S und einem Ist-Datensatz DS-I. Beispielsweise umfasst der Datensatz DS-S eine Darstellung des Zahns in einer vorgegebenen Perspektive. Demgegenüber umfasst der Datensatz DS-I, der aus dem Zahnmodell 200 gerendert worden ist, eine Darstellung des Zahnmodells 200 in der gleichen Perspektive. Die beiden Abbildungen aus dem Soll-Datensatz DS-S und dem Ist-Datensatz DS-I werden auf die gleiche Größe skaliert und nebeneinandergesetzt.

Die euklidische Abweichung $\Delta E_{S,\,I}$ zwischen dem Soll-Datensatz DS-S und dem Ist-Datensatz DS-I kann berechnet werden, indem die Unterschiede in den Farbwerten der Pixel entlang der Vergleichslinien 207 aufsummiert werden, beispielsweise im L*a*b*-Farbraum. Diese Vergleichslinien 207 können beliebig verschoben werden, indem beispielsweise die Zahnhälften zusammengeschoben werden. Im Allgemeinen kann die Vergleichslinie 207 jedoch auch einen anderen Verlauf aufweisen. Der Vergleich kann auf Pixel-Ebene als kleinste Auflösung erfolgen.

$$\Delta E_{S,I} = \sqrt{(L_S^* - L_I^*)^2 + (a_S^* - a_I^*)^2 + (b_S^* - b_I^*)^2}$$

Je größer der Unterschied im Farbverlauf entlang der Vergleichslinie 207 ist, desto größer ist die numerische Abweichung $\Delta E_{S,\,I}$. Bei vollkommener farblicher Übereinstimmung zwischen dem Soll-Datensatz DS-S und dem Ist-Datensatz DS-I ist die Abweichung $\Delta E_{S,\,I}$ Null. Im Allgemeinen können jedoch auch andere Verfahren zum Berechnen der Abweichung $\Delta E_{S,\,I}$ herangezogen werden, wie beispielsweise auf der Basis von Spektralinformation.

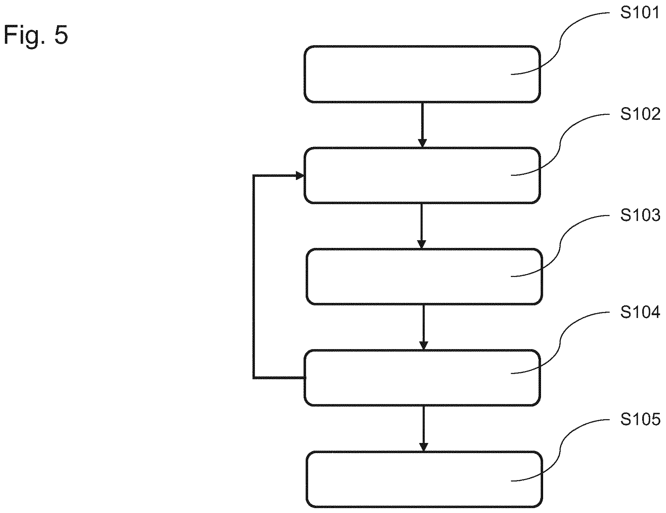

Fig. 5 zeigt ein Blockdiagramm eines Verfahrens zum Herstellen der dentalen Restauration 100. Zunächst wird in Schritt S101 das digitale Zahnmodell 200 mit dem ersten räumlichen Bereich 203-1, in dem ein erstes Restaurationsmaterial 201-1 angeordnet ist, und dem zweiten räumlichen Bereich 203-2 erzeugt, in dem in zweites Restaurationsmaterial 201-2 angeordnet ist. Dieses Zahnmodell 200 dient als Startpunkt für das Verfahren. In Schritt S102 wird das anfängliche digitale Zahnmodell 200 zum Erzeugen des Ist-Datensatzes DS-I gerendert, der die optischen Eigenschaften des digitalen Zahnmodells 200 wiedergibt. Die Berechnung und Simulation des Zahnmodells 200 erfolgt basierend auf physikalisch-optisch materialspezifischen Kennwerten mittels eines physikalisch korrekten Simulationstools. In Schritt S103 wird die Abweichung zwischen dem Soll-Datensatz DS-S und dem Ist-Datensatz DS-I bestimmt.

In Schritt S104 wird der erste räumliche Bereich 203-1 und/oder der zweite räumliche Bereichs 203-2 verändert, um eine geringere Abweichung $\Delta E_{S,\,I}$ zwischen dem erfassten Soll-Datensatz DS-S und dem Ist-Datensatz DS-I des erneut gerenderten digitalen Zahnmodells 200 zu erhalten. Zu diesem Zweck werden der Schritt S103 des Renderns des Zahnmodells 200 mit den verformten Bereichen 203 und der Schritt S104 des Berechnens einer Abweichung zwischen dem Soll-Datensatz DS-S und dem Ist-Datensatz DS-I erneut durchgeführt. Ist beispielsweise ein vorgegebener Grenzwert für die Abweichung unterschritten, wird die innere Formgebung des Zahnmodells 200 mit den Restaurationsmaterialien 201-1, ..., 201-3 eingefroren. In Schritt S105 wird dann die dentale Restauration 100 auf Basis des ermittelten digitalen Zahnmodells 200 mit der geringeren Abweichung hergestellt. Die dentale Restauration 100 kann durch ein 3D-Druckverfahren oder ein anderes geeignetes Verfahren auf Basis des Zahnmodells 200 mit den berechneten räumlichen Bereichen und den jeweiligen Restaurationsmaterialien 201-1, ..., 201-3 hergestellt werden.

In diesem Fall kann eine Computervorrichtung verwendet werden, die die Berechnungsschritte ausführt und anschließend die dentale Restauration 100 mittels der Herstellungsvorrichtung herstellt. Die Computervorrichtung führt zu diesem Zweck ein Computerprogramm aus, das Befehle umfasst, die bewirken, dass die Computervorrichtung die erforderlichen Verfahrensschritte ausführt. Die Computervorrichtung umfasst einen Prozessor und einen digitalen Speicher, in dem die Datensätze und ein Computerprogramm gespeichert ist, das die Verfahrensschritte ausführt und eine Herstellungsvorrichtung geeignet ansteuert. Die Herstellungsvorrichtung ist beispielsweise ein 3D-Drucker, der die dentale Restauration 100 mit den unterschiedlichen Restaurationsmaterialien druckt. Im Allgemeinen können jedoch auch andere Herstellungsvorrichtungen verwendet werden, die die dentale Restauration mit den unterschiedlichen Restaurationsmaterialien herstellen kann.

Nach einem Einsetzen der so erzeugten dentalen Restauration 100 ist das optische Erscheinungsbild des biomimetischen Zahnersatzes optimal an das gewünschte natürliche Erscheinungsbild angenähert.

Bei dem Verfahren handelt es sich um einen iterativen, geschlossenen digitalen Prozess, bei dem sich eine Modellierung und Simulation der dentalen Restauration 100 soweit wie möglich an das natürliche Erscheinungsbild im Patientenmund annähert. Weil mit den physikalischen und optischen Parametern bekannter Restaurationsmaterialien 201-1, ..., 201-3 in den räumlichen Bereichen 203-1, 203-2 und 203-3 simuliert wird, kann ein rechnergestütztes naturgetreues Erscheinungsbild der dentalen Restauration 100 gewonnen werden.

Durch das Herstellungsverfahren kann eine optimale Gestaltung des inneren mehrschichtigen Aufbaus des Zahnmodels 200 erreicht werden und gleichzeitig eine optimale Ästhetik der späteren dentalen Restauration 100 sichergestellt werden. Hierzu ist es möglich, eine optimale Dicke und räumliche Lage der einzelnen räumlichen Bereiche 203-11, ..., 203-3 zu finden. Die Restaurationsmaterialien 201-1, ..., 201-3 zur Erzeugung der mehrschichtigen dentalen Restauration 100 für die Fertigung der dentalen Restauration 100 und deren Anordnung sind bekannt.

Bei dem Verfahren handelt es sich somit um einen iterativen Prozess, bei dem automatisch eine optimierte innere Architektur einer dentalen Restauration 100 erzeugt wird. Dies kann unabhängig von einer ersten strukturellen Vorgabe für das Zahnmodell 200 erreicht werden. Durch die Übereinstimmung zwischen dem Ist-Datensatz und dem Soll-Datensatz (Best Match) wird ein wiederholbares optimales Ergebnis mit den zur Verfügung stehenden Restaurationsmaterialien 201-1, ..., 201-3 und eine optimale Ästhetik der dentalen Restauration 100 erreicht. Subjektive Einflüsse des Anwenders auf den optischen Gesamteindruck können hierbei ausgeschlossen werden. Eine objektive Genauigkeit und Wiederholbarkeit des Verfahrens schließen Fehler durch eine räumliche Gestaltung eines Benutzers aus.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

BEZUGSZEICHENLISTE

| | |
|---|---|
| 100 | Dentale Restauration |
| 101 | elektronische Kamera |

| | |
|---|---|
| 200 | Zahnmodell |
| 201 | Restaurationsmaterial |
| 203 | räumlicher Bereich/Schicht |
| 205 | Grenzfläche |
| 207 | Vergleichslinie |
| 209 | Zahnstumpf |
| 211 | Zahnbogen |
| 213 | Mittelebene |

| | |
|---|---|
| DS-I | Ist-Datensatz |
| DS-S | Soll Datensatz |

**Patentansprüche**

1. Verfahren zum Herstellen einer dentalen Restauration (100), mit den Schritten:

   - Erzeugen (S101) eines digitalen Zahnmodells (200) mit einem ersten räumlichen Bereich (203-1), in dem ein erstes Restaurationsmaterial (201-1) angeordnet ist, und einem zweiten räumlichen Bereich (203-2), in dem ein zweites Restaurationsmaterial (201-2) angeordnet ist;
   - Rendern (S102) des digitalen Zahnmodells (200) zum Erzeugen eines Ist-Datensatzes (DS-I), der die optischen Eigenschaften des digitalen Zahnmodells (200) wiedergibt;
   - Bestimmen (S103) einer Abweichung ($\Delta E\,S,I$) zwischen einem erfassten Soll-Datensatz (DS-S) und dem Ist-Datensatz (DS-I);
   - Verändern (S104) des ersten räumlichen Bereichs (203-1) und/oder zweiten räumlichen Bereichs (203-2), um eine geringere Abweichung ($\Delta E\,S,I$) zwischen dem erfassten Soll-Datensatz (DS-S) und dem Ist-Datensatz (DS-I) des erneut gerenderten digitalen Zahnmodells (200) zu erhalten; wobei der erste räumliche Bereich (203-1) und/oder zweite räumliche Bereich (203-2) solange verformt wird, bis die Abweichung unter einem vorgegebenen Wert liegt; und
   - Herstellen (S105) der dentalen Restauration (100) auf Basis des digitalen Zahnmodells (200) mit der geringeren Abweichung.

2. Verfahren nach Anspruch 1, wobei das Verändern (S104) derart erfolgt, dass der erste räumliche Bereich ein äußerer Bereich des Zahnmodells (200) ist, der nach innen verformt wird.

3. Verfahren nach einem der vorangehenden Ansprüche, wobei das Verändern (S104) derart erfolgt, dass der zweite räumliche Bereich ein innerer Bereich des Zahnmodells (200) ist, der nach außen verformt wird.

4. Verfahren nach einem der vorangehenden Ansprüche, wobei das digitale Zahnmodell (200) mit einer Grenzfläche zwischen dem ersten räumlichen Bereich (203-1) und dem zweiten räumlichen Bereich (203-2) erzeugt wird.

5. Verfahren nach Anspruch 4, wobei die Grenzfläche in einer Mitte des digitalen Zahnmodells (200) liegt.

6.  Verfahren nach Anspruch 5, wobei die Grenzfläche (205) tangential zu einem Zahnbogen (211) an der entsprechenden Zahnposition verläuft.

7.  Verfahren nach Anspruch 4, wobei die Grenzfläche an einer Innenseite oder einer Außenseite des digitalen Zahnmodells (200) liegt.

8.  Verfahren nach Anspruch 7, wobei durch die Grenzfläche (205) ein Keimbereich gebildet wird, von dem ausgehend der erste räumliche Bereich (203-1) und/oder der zweite räumliche Bereich (203-2) verändert werden.

9.  Verfahren nach einem der vorangehenden Ansprüche, wobei der erste räumliche Bereich (203-1) und der zweite räumliche Bereich (203-2) schichtweise zueinander angeordnet sind.

10. Verfahren nach einem der vorangehenden Ansprüche, wobei der erste räumliche Bereich (203-1) und/oder zweite räumliche Bereich (203-2) solange verändert wird, bis die Abweichung ein Minimum erreicht hat.

11. Verfahren nach einem der vorangehenden Ansprüche, wobei der Soll-Datensatz (DS-S) auf Basis eines natürlichen Zahns erhalten wird.

12. Verfahren nach Anspruch 11, wobei der Soll-Datensatz (DS-S) die optischen Eigenschaften und/oder die Geometrie des natürlichen Zahns wiedergibt.

13. Computervorrichtung zum Herstellen einer dentalen Restauration (100), mit einer Herstellungsvorrichtung, die geeignet ist, das Verfahren nach einem der Ansprüche 1 bis 12 auszuführen.

14. Computerprogramm, umfassend Befehle, die bewirken, dass die Computervorrichtung des Anspruchs 13 die Verfahrensschritte nach einem der Ansprüche 1 bis 12 ausführt.

**Claims**

1.  A method of producing a dental restoration (100), comprising the steps of:

    - generating (S101) a digital tooth model (200) having a first spatial region (203-1) in which a first restoration material (201-1) is arranged and a second spatial region (203-2) in which a second restoration material (201-2) is arranged;
    - rendering (S102) the digital tooth model (200) to generate an actual data set (DS-I) representing the optical properties of the digital tooth model (200);
    - determining (S103) a deviation ($\Delta E_{S,I}$) between an acquired target data set (DS-S) and the actual data set (DS-I);
    - altering (S104) the first spatial region (203-1) and/or second spatial region (203-2) to obtain a smaller deviation ($\Delta E_{S,I}$) between the acquired target data set (DS-S) and the actual data set (DS-I) of the re-rendered digital tooth model (200); wherein the first spatial region (203-1) and/or second spatial region (203-2) is deformed until the deviation is below a predetermined value; and
    - producing (S105) the dental restoration (100) based on the digital tooth model (200) with the smaller deviation.

2.  The method according to claim 1, wherein the altering (S104) is performed such that the first spatial region is an outer region of the tooth model (200) that is deformed inwardly.

3.  The method according to any one of the preceding claims, wherein the altering (S104) is performed such that the second spatial region is an inner region of the tooth model (200) that is deformed outwardly.

4.  The method according to any one of the preceding claims, wherein the digital tooth model (200) is generated with a boundary surface between the first spatial region (203-1) and the second spatial region (203-2).

5.  The method according to claim 4, wherein the boundary surface is located in a center of the digital tooth model (200) .

6.  The method according to claim 5, wherein the boundary surface (205) extends tangential to a dental arch (211) at the corresponding tooth position.

7.  The method according to claim 4, wherein the boundary surface is located on an inner side or an outer side of the digital tooth model (200).

8.  The method according to claim 7, wherein a nucleation region is formed by the boundary surface (205), starting from which the first spatial region (203-1) and/or the second spatial region (203-2) are altered.

9.  The method according to any one of the preceding claims, wherein the first spatial region (203-1) and the second spatial region (203-2) are arranged in layers relative to each other.

10. The method according to any one of the preceding claims, wherein the first spatial region (203-1) and/or second spatial region (203-2) is altered until the deviation has reached a minimum.

11. The method according to any one of the preceding claims, wherein the target data set (DS-S) is obtained based on a natural tooth.

12. The method according to claim 11, wherein the target data set (DS-S) represents the optical properties and/or the geometry of the natural tooth.

13. A computer device for producing a dental restoration (100), comprising a production device suitable to perform the method of any one of claims 1 to 12.

14. A computer program comprising instructions that cause the computer device of claim 13 to perform the method steps of any one of claims 1 to 12.

**Revendications**

1.  Procédure de réalisation d'une restauration dentaire (100), comprenant les étapes suivantes :

    - la création (S101) d'un modèle dentaire numérique (200) ayant une première région spatiale (203-1) dans laquelle un premier matériau de restauration (201-1) est disposé et une deuxième région spatiale (203-2) dans laquelle un second matériau de restauration (201-2) est disposé ;
    - le rendu (S102) du modèle numérique dentaire (200) pour générer un ensemble de données réelles (DS-I) reflétant les propriétés optiques du modèle numérique dentaire (200) ;
    - la détermination (S103) d'un écart (AE S,I) entre un ensemble de données cible enregistré (DS-S) et l'ensemble de données réel (DS-I) ;
    - la modification (S104) de la première région spatiale (203-1) et/ou la deuxième région spatiale (203-2) afin d'obtenir une variance plus petite (AE S,I) entre l'ensemble de données cible acquis (DS-S) et l'ensemble de données réel (DS-I) du modèle dentaire numérique (200) rendu à nouveau; où la première région spatiale (203-1) et/ou la deuxième région spatiale (203-2) est déformée jusqu'à ce que l'écart soit inférieur à une valeur prédéterminée ; et
    - la fabrication (S105) de la restauration dentaire (100) sur la base du modèle dentaire numérique (200) ayant l'écart le plus faible.

2.  Procédé selon la revendication 1, où la modification (S104) est réalisée de telle sorte que la première région spatiale est une région externe du modèle dentaire (200) qui est déformée vers l'intérieur.

3.  Procédé selon l'une des revendications précédentes, où la modification (S104) est réalisée de telle sorte que la deuxième région spatiale est une région interne du modèle dentaire (200) qui est déformée vers l'extérieur.

4.  Procédé selon l'une des revendications précédentes, où le modèle numérique dentaire (200) est généré avec une interface entre la première région spatiale (203-1) et la deuxième région spatiale (203-2).

5.  Procédé selon la revendication 4, où l'interface se trouve en un centre du modèle dentaire numérique (200).

6.  Procédé selon la revendication 5, où l'interface (205) se trouve tangentielle à une arcade dentaire (211) à la position de dent correspondante.

**7.** Procédé selon la revendication 4, où l'interface est située sur un côté intérieur ou un côté extérieur du modèle dentaire numérique (200).

**8.** Procédé selon la revendication 7, où une région germinale est formée par l'interface (205) à partir de laquelle la première région spatiale (203-1) et/ou la deuxième région spatiale (203-2) sont modifiées.

**9.** Procédé selon l'une des revendications précédentes, où la première région spatiale (203-1) et la deuxième région spatiale (203-2) sont disposées en couches l'une par rapport à l'autre.

**10.** Procédé selon l'une quelconque des revendications précédentes, où la première région spatiale (203-1) et/ou la deuxième région spatiale (203-2) est modifiée jusqu'à ce que l'écart atteigne un minimum.

**11.** Procédé selon l'une quelconque des revendications précédentes, où l'ensemble de données cible (DS-S) est obtenu sur la base d'une dent naturelle.

**12.** Procédé selon la revendication 11, où l'ensemble de données cible (DS-S) reflète les propriétés optiques et/ou la géométrie de la dent naturelle.

**13.** Dispositif informatique pour la fabrication d'une restauration dentaire (100), comprenant un dispositif de fabrication apte à mettre en œuvre le procédé selon l'une des revendications 1 à 12.

**14.** Programme informatique, comprenant des commandes qui font que le dispositif informatique de la revendication 13 exécute les étapes du procédé selon l'une quelconque des revendications 1 à 12.

Fig. 1

EP 3 984 494 B1

Fig. 2

EP 3 984 494 B1

Fig. 3

Fig. 4

EP 3 984 494 B1

Fig. 5

S101

S102

S103

S104

S105

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2019023461 A1

- US 2012205828 A1